(19) Europäisches Patentamt
European Patent Office
Office européen des brevets

(11) Veröffentlichungsnummer: **0 274 023 A1**

(12) # EUROPÄISCHE PATENTANMELDUNG

(21) Anmeldenummer: **87116500.7**

(22) Anmeldetag: **09.11.87**

(51) Int. Cl.⁴: **C07C 87/44**, C07D 295/02, C07D 275/06, A01N 43/80

(30) Priorität: **22.11.86 DE 3639901**

(43) Veröffentlichungstag der Anmeldung: **13.07.88 Patentblatt 88/28**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Weissmüller, Joachim, Dr.**
**Carl-Langhans-Strasse 53**
**D-4019 Monheim(DE)**
Erfinder: **Dutzmann, Stefan, Dr.**
**Leinenweberweg 33**
**D-4000 Düsseldorf 13(DE)**
Erfinder: **Reinecke, Paul, Dr.**
**Steinstrasse 8**
**D-5090 Leverkusen 3(DE)**

(54) **Saccharin-Salze von substituierten Aminen.**

(57) Saccharin-Salze von substituierten Aminen der allgemeinen Formel (I),

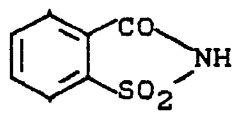 x 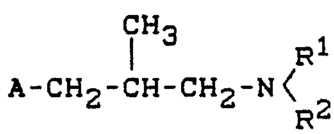 (I)

in welcher
A für jeweils gegebenenfalls substituiertes 2-Decahydronaphthyl oder β-Naphthyl steht und
$R^1$ und $R^2$ unabhängig voneinander jeweils für Alkyl oder Alkenyl stehen oder gemeinsam mit dem Stickstoffatom, an welches sie gebunden sind, für einen gegebenenfalls substituierten, gesättigten Heterocyclus stehen, der weitere Heteroatome enthalten kann,
und ihre Verwendung zur Bekämpfung von Schädlingen.

**EP 0 274 023 A1**

## Saccharin-Salze von substituierten Aminen

Die Erfindung betrifft neue Saccharin-Salze von substituierten Aminen, ein Verfahren zu ihrer Herstellung sowie ihre Verwendung als Schädlingsbekämpfungsmittel.

Es ist bereits bekannt, daß Saccharin-Salze von Aminen, wie beispielsweise das Saccharin-Salz des 5-Amino-1,2,4-triazols oder das Saccharin-Salz des Cyclohexylamins fungizide Eigenschaften besitzen (vgl. EP 158 074).

Die Wirkung dieser vorbekannten Verbindungen ist jedoch insbesondere bei niedrigen Aufwandmengen und Konzentrationen nicht in allen Anwendungsgebieten völlig zufriedenstellend.

Es wurden neue Saccharin-Salze von substituierten Aminen der allgemeinen Formel (I),

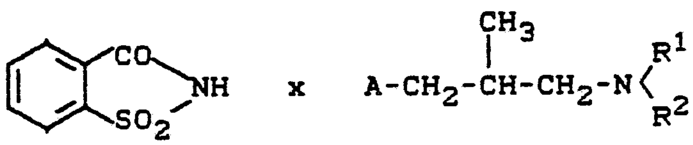
(I)

in welcher
A für jeweils gegebenenfalls substituiertes 2-Decahydronaphthyl oder $\beta$-Naphthyl steht und
$R^1$ und $R^2$ unabhängig voneinader jeweils für Alkyl oder Alkenyl stehen oder gemeinsam mit dem Stickstoffatom, an welches sie gebunden sind, für einen gegebenenfalls substituierten, gesättigten Heterocyclus stehen, der weitere Heteroatome enthalten kann,
gefunden.

Die Verbindungen der Formel (I) können als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen Saccharin-Salze von substituierten Aminen der allgemeinen Formel (I),

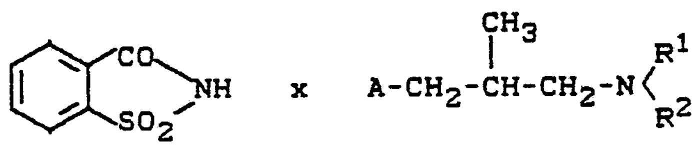
(I)

in welcher
A für jeweils gegebenenfalls substituiertes 2-Decahydronaphthyl oder $\beta$-Naphthyl steht und
$R^1$ und $R^2$ unabhängig voneinander jeweils für Alkyl oder Alkenyl stehen oder gemeinsam mit dem Stickstoffatom, an welches sie gebunden sind, für einen gegebenenfalls substituierten, gesättigten Heterocyclus stehen, der weitere Heteroatome enthalten kann,
erhält, wenn man substituierte Amine der Formel (II),

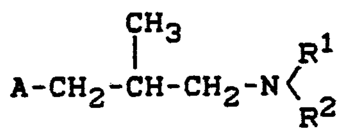
(II)

in welcher
A, $R^1$ und $R^2$ die oben angegebene Bedeutung haben,
mit Saccharin gegebenenfalls in Gegenwart eines Verdünnungsmittels umsetzt.

Schließlich wurde gefunden, daß die neuen Saccharin-Salze von substituierten Aminen der allgemeinen Formel (I) eine Wirkung gegen Schädlinge besitzen.

Überraschenderweise zeigen die erfindungsgemäßen Saccharin-Salze von substituierten Aminen der allgemeinen Formel (I) z.B. eine bessere fungizide Wirksamkeit als die aus dem Stand der Technik bekannten Saccharin-Salze von Aminen, wie beispielsweise das Saccharin-Salz des 5-Amino-1,2,4-triazols oder das Saccharin-Salz des Cyclohexylamins, welches chemisch und wirkungsmäßig naheliegende Verbindungen sind.

Die erfindungsgemäßen Saccharin-Salze von substituierten Aminen sind durch die Formel (I) allgemein definiert. Bevorzugt sind Salze der Formel (I), bei welchen

A für gegebenenfalls ein-bis dreifach, gleich oder verschieden substituiertes 2-Decahydronaphthyl steht, wobei als Substituenten genannt seien: Hydroxy, jeweils geradkettiges oder verzweigtes Alkyl oder Alkoxy mit jeweils 1 bis 6 Kohlenstoffatomen; außerdem für gegebenenfalls ein-bis dreifach, gleich oder verschieden substituiertes $\beta$-Naphthyl steht, wobei als Substituenten genannt seien: Halogen, Hydroxy, jeweils geradkettiges oder verzweigtes Alkyl oder Alkoxy mit jeweils 1 bis 6 Kohlenstoffatomen,

$R^1$ und $R^2$ unabhängig voneinander für jeweils geradkettiges oder verzweigtes Alkyl oder Alkenyl mit jeweils bis zu 6 Kohlenstoffatome stehen oder gemeinsam mit dem Stickstoffatom, an welches sie gebunden sind, für einen gegebenenfalls ein-bis dreifach, gleich oder verschieden substituierten, gesättigten fünf-bis siebengliedrigen Heterocyclus stehen, der 1 oder 2 weitere Heteroatome, insbesondere Stickstoff oder Sauerstoff enthalten kann, wobei als Substituenten jeweils genannt seien; jeweils geradkettiges oder verzweigtes Alkyl oder Hydroxyalkyl mit jeweils 1 bis 4 Kohlenstoffatomen.

Besonders bevorzugt sind Salze der Formel (I), bei welchen

A für gegebenenfalls ein-oder zweifach, gleich oder verschieden substituiertes 2-Decahydronaphthyl steht, wobei als Substituenten jeweils genannt seien: Methyl, Ethyl, n-oder i-Propyl, n-i-, s-, oder t-Butyl, 2-Methylbut-2-yl, Methoxy oder Ethoxy; außerdem für gegebenenfalls ein-bis dreifach, gleich oder verschieden substituiertes $\beta$-Naphthyl steht, wobei als Substituenten genannt seien: Fluor, Chlor, Brom, Methyl, Ethyl, n- oder i-Propyl, n-, i-, s-oder t-Butyl, 2-Methyl-but-2-yl, Methoxy oder Ethoxy,

$R^1$ und $R^2$ unabhängig voneinander jeweils für Methyl, Ethyl, n-oder i-Propyl, Allyl, Butenyl, Dimethylallyl, n-oder i-Butyl, n-oder i-Pentenyl stehen oder gemeinsam mit dem Stickstoffatom, an welches sie gebunden sind, für einen gegebenenfalls ein-bis dreifach, gleich oder verschieden substituierten Heterocyclus der Formel

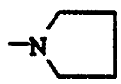 , 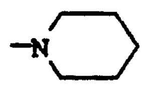 , 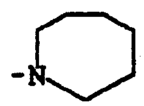 oder 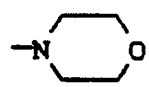

stehen, wobei als Substituenten in Frage kommen: Methyl, Ethyl oder Hydroxymethyl.

Im einzelnen seien außer den bei den Herstellungsbeispielen genannten Verbindungen die folgenden Salze der allgemeinen Formel (I) genannt:

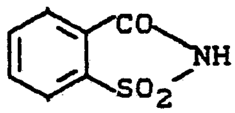 x 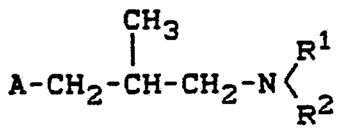

| A | 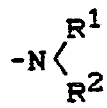 |
|---|---|
| 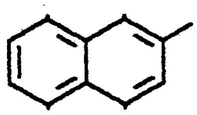 | 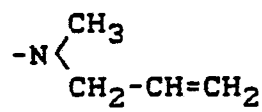 |
| 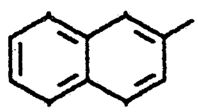 | 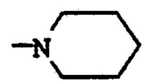 |
| 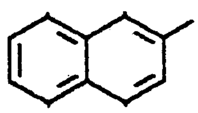 | 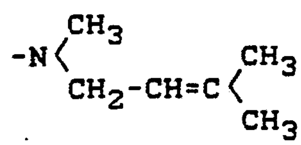 |
| 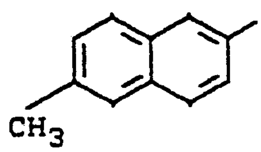 | 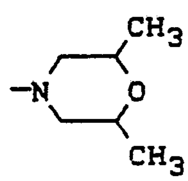 |

| A | 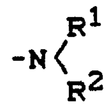 |
|---|---|
| 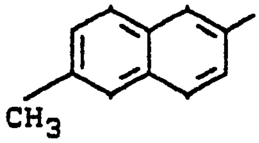 | 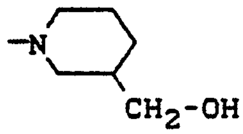 |
| 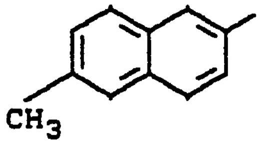 | 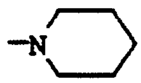 |
| 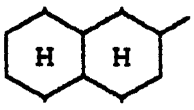 | 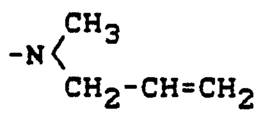 |
| 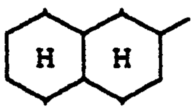 | 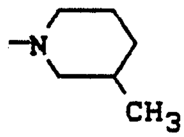 |
| 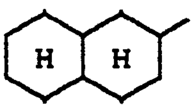 | 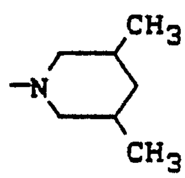 |
| 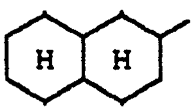 | 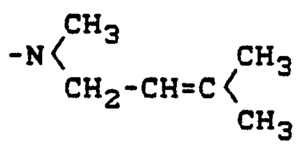 |
| 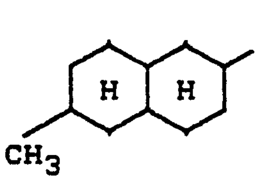 | 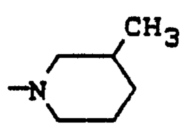 |
| 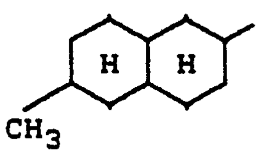 | 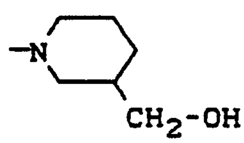 |

| A | 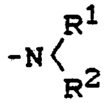 |
|---|---|
| 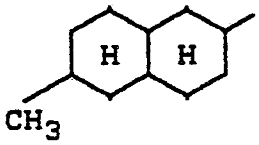 | 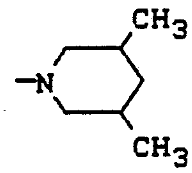 |
| 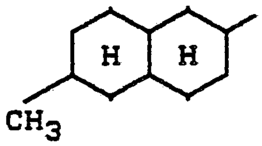 | 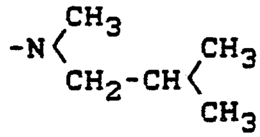 |
| 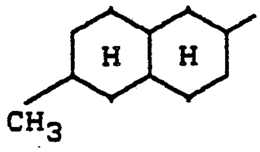 | 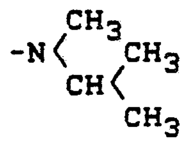 |
| 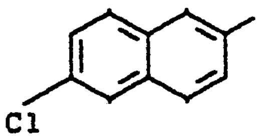 | 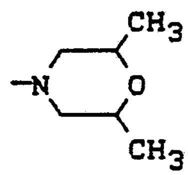 |
| 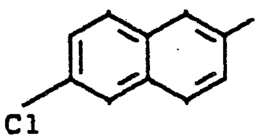 | 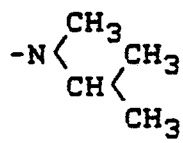 |

Verwendet man beispielsweise 1-β-Naphthyl-2-methyl-3-(3-methylpiperidin-1-yl)-propan und Saccharin als Ausgangsstoffe, so läßt sich der Reaktionsablauf des erfindungsgemäßen Verfahrens durch das folgende Formelschema darstellen:

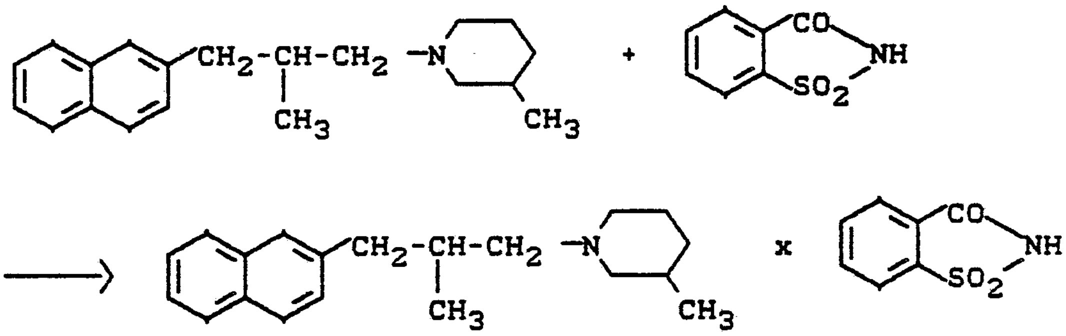

Die zur Durchführung des erfindungsgemäße Verfahrens als Ausgangsstoffe benötigten substituierten Amine sind durch die Formel (II) allgemein definiert. In dieser Formel (II) stehen A, $R^1$ und $R^2$ für diejenigen Reste, die bereits im Zusammenhang mit der Beschreibung der erfindungsgemäßen Stoffe der Formel (I) für diese Substituenten genannt wurden.

Die substituierten Amine der Formel (II) sind teilweise bekannt (vgl. DE-OS 34 13 897).

Noch nicht bekannt sind substituierte Amine der Formel (IIa),

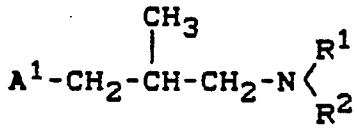 (IIa)

in welcher
$A^1$ für gegebenenfalls substituiertes 2-Dechaydronaphthyl steht und
$R^1$ und $R^2$ die oben angegebene Bedeutung haben.

Sie sind jedoch Gegenstand einer eigenen noch nicht publizierten Patentanmeldung (vgl. Deutsche Patentanmeldung P 3 624 648 vom 22.07.1986) und erhältlich, beispielsweise indem man die bekannten substituierten Amine der Formel (IIb),

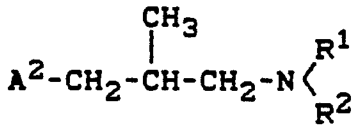 (IIb)

in welcher
$A^2$ für gegebenenfalls substituiertes $\beta$-Naphthyl steht und
$R^1$ und $R^2$ die oben angegebene Bedeutung haben,
in üblicher Art und Weise mit Wasserstoff in Gegenwart eines Katalysators, wie beispielsweise Ruthenium auf Kohlenstoff, gegebenenfalls in Gegenwart eines Verdünnungsmittels, wie beispielsweise Tetrahydrofuran, in einem Druckbereich zwischen 1 und 300 atm und bei einer Temperatur zwischen 20 °C und 250 °C hydriert.

Als Verdünnungsmittel zur Durchführung des erfindungsgemäßen Verfahrens kommen inerte organische Lösungsmittel infrage.

Hierzu gehören insbesondere Ether wie Diethylether, Dioxan, Tetrahydrofuran oder Ethylenglykoldimethyl-oder -diethylether; Ketone wie Aceton oder Butanon; Nitrile wie Acetonitril oder Propionitril; Amide wie Dimethylformamid, Dimethylacetamid, N-Methylformanilid, N-Methylpyrrolidon oder Hexamethylphosporsäuretriamid oder Alkohole wie Methanol, Ethanol oder Propanol.

Die Reaktionstemperaturen können bei der Durchführung des erfindungsgemäßen Verfahrens in einem größeren Bereich variiert werden. Im allgemeinen arbeitet man bei Temperaturen zwischen 0 °C und 100 °C, vorzugsweise bei Temperaturen zwischen 10 °C und 50 °C.

Zur Durchführung des erfindungsgemäßen Verfahrens setzt man pro Mol an substituierten Amin der Formel (II) äquimolare Mengen an Saccharin zu. Man löst beide Reaktionspartner bei der geeigneten Reaktionstemperatur in einem geeigneten Lösungsmittel und entfernt anschließend das Lösungsmittel durch Destillation in Vakuum. Die so erhältlichen Salze, die gelegentlich als zähe Öle oder amorph anfallen, können nach allgemein üblichen Verfahren, wie z.B. durch Umkristallisieren oder Digerieren in geeigneten Lösungsmitteln, gereinigt werden. Die Charakterisierung erfolgt in diesen Fällen mit Hilfe spektroskopischer Verfahren (IR; NMR).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;

Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wei beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis), gegen den Erreger der Blattfleckenkrankheit an Getreide (Pyrenophora teres), gegen den Erreger der Braunfleckigkeit des Weizens (Septoria nodorum), gegen Oomyceten-Arten und gegen den Erreger der Reisfleckenkrankeit (Pyricularia oryzae) einsetzen. Hervorzuheben ist, daß die erfindungsgemäßen Wirkstoffe neben einer guten protektiven Wirksamkeit auch systemische Eigenschaften besitzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck ste henden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoff sowie Butan, Propan, Stickstoff und Kohlendioxid; als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und orga nischen Mehlen sowie Granulate aus organischem Material wie Sägemehl, Kokosnußschalen, Maiskolben und Tabakstengel; als Emulgier-und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phosphlipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulie rungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

### Beispiel 1:

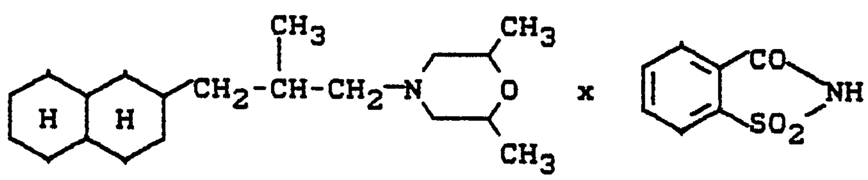

4,5 g (0,014 Mol) 1-(2,6-Dimethylmorpholin-4-yl)-2-methyl-3-(decahydronaphth-2-yl)-propan und 2,58 g (0,014 Mol) Saccharin werden zusammen in 80 ml Aceton gelöst und 15 Minuten bei Raumtemperatur gerührt. Nach Verdampfen des Lösungsmittels erhält man 7 g (100 % der Theorie) an 1-(2,6-Dimethylmorpholin-4-yl)-2-methyl-3-(decahydronaphth-2-yl)-propan Saccharin-Salz als amorphen Feststoff.

$^1$H-NMR: $\delta$ = 7,6 (m,2H); 7,8 (m,2H); 4,2-4,4 (m,2H); 3,55-3,75 (m,2H); 3,0-3,1 (m,1H); 2,85-2,95 (m,1H); 2,3-2,5(m,2H); 2,1-2,2 (m,1H); 0,8-1,8 (m,28H) ppm.

### Beispiel 2:

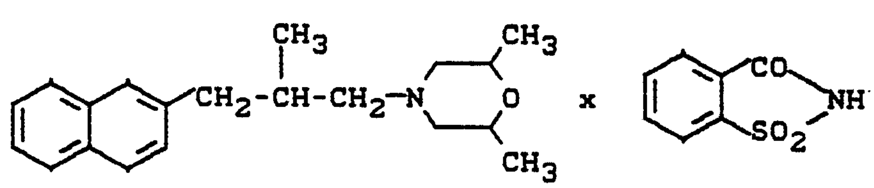

Zu einer Lösung von 5,24 g (0,0176 Mol) 1-(2,6-Dimethylmorpholin-4-yl)-2-methyl-3-$\beta$-naphthyl-propan in 40 ml Ethanol gibt man eine Lösung von 3,23 g (0,0176 Mol) Saccharin in 50 ml Ethanol rührt 10 Minuten bei Raumtemperatur und entfernt das Lösungsmittel im Vakuum.

Man erhält 8,47 g (100% der Theorie) an 1-(2,6-Dimethylmorpholin-4-yl)-2-methyl-3-$\beta$-naphthyl-propan

Saccharin-Salz als amorphen Feststoff.

$^1$H-NMR, δ = 7.2-8.0 (m,11H); 4.2-4.4 (m,2H); 3.4-3.7 (m,2H); 3.1-3.2 (m,1H); 2.8-3.0 (m,2H); 2.1-2.6 (m,2H); 0,6-1.6 (m,12H) ppm

Herstellung der Ausgangsverbindungen

1a) 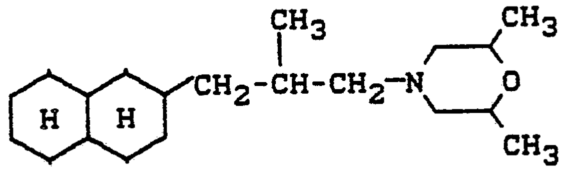

10 g (0,033 Mol) 1-(2,6-Dimethylmorpholin-4-yl)-2-methyl-3-β-naphthyl-propan werden in 150 ml Tetrahydrofuran bei 120° in Gegenwart von 3 g Ruthenium-Kohlenstoff (5 %) und bei einem Wasserstoffdruck von 150 bar 3 Stunden hydriert. Vom Katalysator wird abfiltriert und das Lösungsmittel im Vakuum verdampft.

Man erhält 7,7 g (74,6 % der Theorie) an 1-(2,6-Dimethylmorpholin-4-yl)-2-methyl-3-(decahydronaphth-2-yl)-propan von Brechungsindex $n_D^{20}$ = 1,4869.

2a) 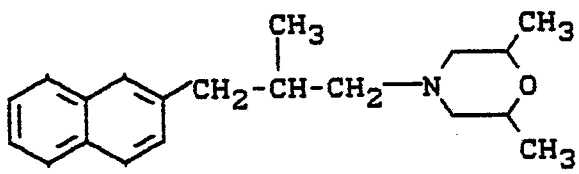

10,8 g (0,04 Mol) 1-Methansulfonyl-2-methyl-3-β-naphthyl-propan und 9 g (0,078 Mol) 2,6-Diemthylmorpholin werden bei einer Badtemperatur von 140°C 15 Stunden gerührt. Die erhaltene Reaktionsmischung wird mit Wasser versetzt und mehrfach mit Ether extrahiert. Die vereinigten organischen Phasen werden über Natriumsulfat getrocknet und im Vakuum von Lösungsmittel befreit; der ölige Rückstand wird säulenchromatographisch (Kieselgel 60, Petrolether/Essigester = 2 : 1) gereinigt.

Man erhält 6,2 g (52 % der Theorie) an 1-(2,6-Dimethylmorpholin-4-yl)-2-methyl-3-β-naphthyl-propan vom Brechungsindex $n_D^{20}$ = 1,5527.

Herstellung des Ausgangsproduktes zur Herstellung von 2a

---

2b) 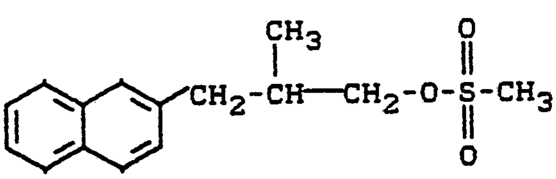

Zu 14 g (0,074 Mol) 2-Methyl-3-β-naphthylpropanol (roh) in 80 ml absolutem Pyridin gibt man bei 0°C tropfenweise unter Rühren 11 g (0,1 Mol) Methansulfonsäurechlorid, rührt nach beendeter Zugabe weitere 16 Stunden bei Raumtemperatur, entfernt überschüssiges Pyridin durch Destillation im Vakuum, nimmt den Rückstand in Wasser auf, extrahiert mehrfach mit Dichlormethan, trocknet über Natriumsulfat und entfernt das Lösungsmittel im Vakuum. Man erhält 13,6 g (66 % der Theorie) an 1-Methansulfonyloxy-2-methyl-3-β-naphthyl-propan als Öl.

(IR: ν = 1345, 1180 $cm^{-1}$).

Herstellung des Ausgangsproduktes zur Herstellung von 2b

2c) 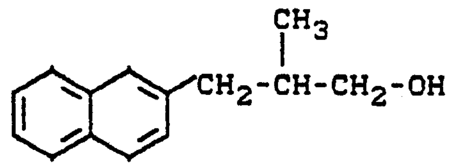

12 g (0,05 Mol) 2-Methyl-3-β-naphthylacrylsäureethylester tropft man unter Eiskühlung in einer trockenen Stickstoffatmosphäre zu einer Suspension von 1,9 g (0,05 Mol) Lithiumaluminiumhydrid in 150 ml absolutem Ether. Nach beendeter Zugabe erwärmt man 8 Stunden lang auf Rückflußtemperatur und tropft dann nach Erkalten der Reaktionsmischung langsam unter Kühlung 15 ml 5-%ige Schwefelsäure zu, saugt den ausgefallenen Feststoff ab, trocknet das Filtrat über Natriumsulfat, entfernt das Lösungsmittel im Vakuum und kristallisiert den Rückstand aus Ether/Petrolether um. Man erhält 7,1 g 2-Methyl-3-β-naphthylpropanol vom Schmelzpunkt 71-74°C, das laut Gaschromatogramm mit 2-Methyl-1-β-naphthyl-propen-3-ol verunreinigt ist und ohne weitere Reinigung in die nächste Reaktionsstufe eingesetzt wird.

Herstellung des Ausgangsproduktes zur Herstellung von 2c

2d) 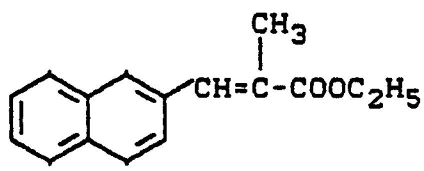

Zu einer Suspension von 5,5 g (0,2 Mol) 80 %igem Natriumhydrid in 300 ml absolutem Xylol gibt man bei 70°C 40 g (0,2 Mol) Ethyl-α-ethoxalylpropionat. Nach beendeter Wasserstoffentwicklung tropft man 31,2 g (0,2 Mol) β-Naphthaldehyd in Xylol gelöst zu und erwärmt nach beendeter Zugabe für 90 Minuten zum Sieden. Die erkaltete Reaktionsmischung wird mit 150 ml Wasser versetzt, die organische Phase abgetrennt, mit 7-%iger Natriumcarbonatlösung gewaschen, über Natriumsulfat getrocknet und eingeengt. Der Rückstand wird im Vakuum destilliert. Man erhält 21,7 g (45, 2 % der Theorie) an 2-Methyl-3-β-naphthylacrylsäureethylester vom Siedepunkt 110°C/0,13 mbar.

In entsprechender Weise und gemäß den allgemeinen Angaben zur Herstellung erhält man die folgenden Saccharin-Salze von substituierten Aminen der allgemeinen Formel (I):

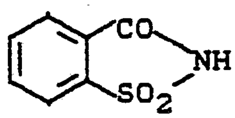 x 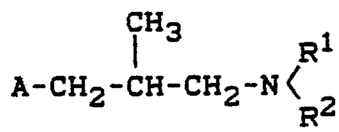 (I)

| Bsp. Nr. | A | 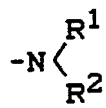 | physikalische Daten |
|---|---|---|---|
| 3 | 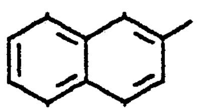 | 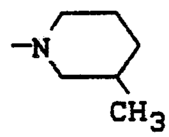 | Fp. 42° C |
| 4 | 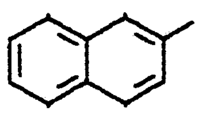 | 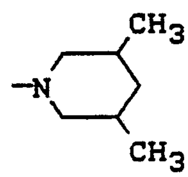 | $^1H$-NMR*): 3,3-3,7; 3,0-3,2; 2,5-3,0; 2,2-2,5; |
| 5 | 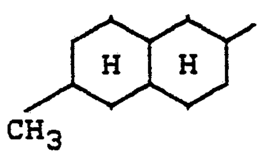 | 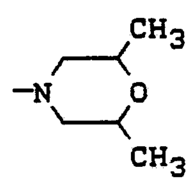 | Fp. 53° C - 56° C |

| Bsp. Nr. | A | 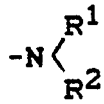 | physikalische Daten |
|---|---|---|---|
| 6 | 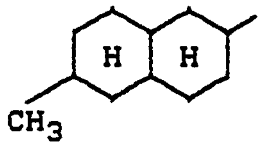 | 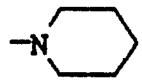 | Fp. 36° C |
| 7 | 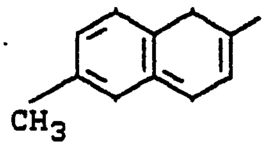 | 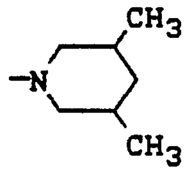 | $^1$H-NMR*): 3,3-3,7; 3,0-3,2; 2,6-3,0; |
| 8 | 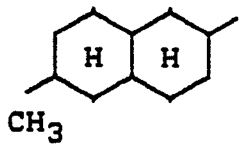 | 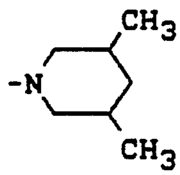 | mp. 34° C |

*) Die $^1$H-NMR-Spektren wurden in $CDCl_3$ mit Tetramethylsilan (TMS) als innerem Standard aufgenommen. Angegeben ist die chemische Verschiebung als δ-Wert in ppm.

Anwendungsbeispiele:

In den folgenden Anwendungsbeispielen wurden die nachstehend aufgeführten Verbindungen als Vergleichsubstanzen eingesetzt:

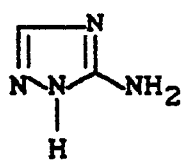 x 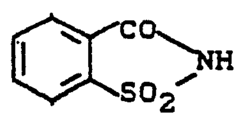 (A)

Saccharin-Salz von 5-Amino-1,2,4-triazol

und

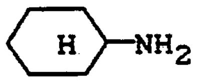 x 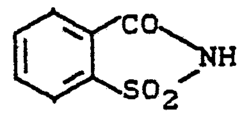 (B)

**Saccharin-Salz von Cyclohexylamin**

(beide bekannt aus EP 158 074).

Beispiel A

Erysiphe-Test (Gerste) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 2, 3 und 4.

**Ansprüche**

1. Saccharin-Salze von substituierten Amine der allgemeinen Formel (I)

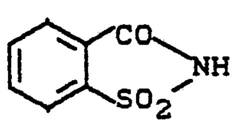 x 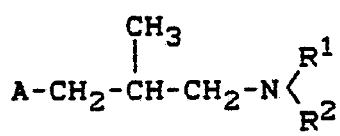 (I)

in welcher

A für jeweils gegebenenfalls substituiertes 2-Decahydronaphthyl oder β-Naphthyl steht und

$R^1$ und $R^2$ unabhängig voneinander jeweils für Alkyl oder Alkenyl stehen oder gemeinsam mit dem Stickstoffatom, an welches sie gebunden sind, für einen gegebenenfalls substituierten, gesättigten Heterocyclus stehen, der weitere Heteroatome enthalten kann.

2. Saccharin-Salze von substituierten Aminen der allgemeinen Formel (I) gemäß Anspruch 1, in welcher A für gegebenenfalls ein-bis dreifach, gleich oder verschieden substituiertes 2-Decahydronaphthyl steht, wobei als Substituenten genannt seien: Hydroxy, jeweils geradkettiges oder verzweigtes Alkyl oder Alkoxy mit jeweils 1 bis 6 Kohlenstoffatomen; außerdem für gegebenenfalls ein-bis dreifach, gleich oder verschieden substituiertes β-Naphthyl steht, wobei als Substituenten genannt seien: Halogen, Hydroxy, jeweils geradkettiges oder verzweigtes Alkyl oder Alkoxy mit jeweils 1 bis 6 Kohlenstoffatomen,

$R^1$ und $R^2$ unabhängig voneinader für jeweils geradkettiges oder verzweigtes Alkyl oder Alkenyl mit jeweils bis zu 6 Kohlenstoffatomen stehen oder gemeinsam mit dem Stickstoffatom, an welches sie gebunden sind, für einen gegebenenfalls ein-bis dreifach, gleich oder verschieden substituierten, gesättigten fünf-bis siebengliedrigen Heterocyclus stehen, der 1 oder 2 weitere Heteroatome enthalten kann, wobei als Substituenten jeweils genannt seien: jeweils geradkettiges oder verzweigtes Alkyl oder Hydroxyalkyl mit jeweils 1 bis 4 Kohlenstoffatome.

3. Saccharin-Salze von substituierten Aminen der allgemeinen Formel (I) gemäß Anspruch 1, in welcher A für gegebenenfalls ein-oder zweifach, gleich oder verschieden substituiertes 2-Decahydronaphthyl steht, wobei als Substituenten genannt seien: Methyl, Ethyl, n-oder i-Propyl, n-i-, s-, oder t-Butyl, 2-Methyl-but-2-yl, Methoxy oder Ethoxy; außerdem für gegebenenfalls ein-bis dreifach, gleich oder verschieden substituiertes β-Naphthyl steht, wobei als Substituenten genannt seien: Fluor, Chlor, Brom, Methyl, Ethyl, n-oder i-Propyl, n-, i-, s-oder t-Butyl, 2-Methyl-but-2-yl, Methoxy oder Ethoxy,
$R^1$ und $R^2$ unabhängig voneinander jeweils für Methyl, Ethyl, n-oder i-Propyl, Allyl, Butenyl, Dimethylallyl, n-oder i-Butyl, n-oder i-Pentenyl stehen oder gemeinsam mit dem Stickstoffatom, an welches sie gebunden sind, für einen gegebenenfalls ein-bis dreifach, gleich oder verschieden substituierten Heterocyclus der Formel

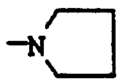 , 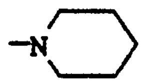 , 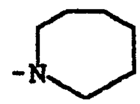 oder 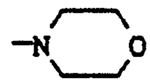

stehen, wobei als Substituenten genannt seien: Methyl, Ethyl oder Hydroxymethyl.

4. Verfahren zur Herstellung von Saccahrin-Salzen von substituierten Aminen der allgemeinen Formel (I)

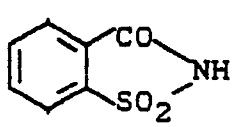 x 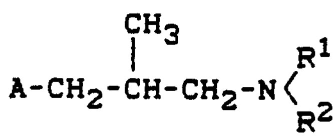 (I)

in welcher
A für jeweils gegebenenfalls substituiertes 2-Decahydronaphthyl oder β-Naphthyl steht und
$R^1$ und $R^2$ unabhängig voneinander jeweils für Alkyl oder Alkenyl stehen oder gemeinsam mit dem Stickstoffatom, an welches sie gebunden sind, für einen gegebenenfalls substituierten, gesättigten Heterocyclus stehen, der weitere Heteroatome enthalten kann,
dadurch gekennzeichnet, daß man substituierte Amine der Formel (II),

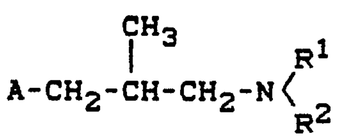 (II)

in welcher
A, $R^1$ und $R^2$ die oben angegebene Bedeutung haben,
mit Saccharin gegebenenfalls in Gegenwart eines Verdünnungsmittels umsetzt.

5. Schädlingsbekämpfungsmittel, gekennzeichnet durch eine Gehalt an mindestens einem Saccharin-Salz von substituierten Aminen der Formel (I) gemäß den Ansprüche 1 und 4.

6. Verwendung von Saccharin-Salzen von Aminen der Formel (I) gemäß den Ansprüchen 1 und 4 zur Bekämpfung von Schädlingen.

7. Verfahren zur Bekämpfung von Schädlingen, dadurch gekennzeichnet, daß man Saccharin-Salze von substituierten Aminen der Formel (I) gemäß den Ansprüchen 1 und 4 auf Schädlinge oder ihren Lebensraum einwirken läßt.

8. Verfahren zur Herstellung von Schädlingsbekämpfungsmitteln, dadurch gekennzeichnet, daß man Saccharin-Salze von substituierten Aminen der Formel (I) gemäß den Ansprüchen 1 und 4 mit Streckmitteln und/oder oberflächenaktiven Mitteln vermischt.

9. Verwendung von Saccharin-Salzen von Aminen der Formel (I) gemäß Anspruch 7 worin die Schädlinge Pilze sind.

Europäisches Patentamt

# EUROPÄISCHER RECHERCHENBERICHT

Nummer der Anmeldung

EP 87 11 6500

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 158 074 (BAYER)<br>* Patentansprüche *<br>--- | 1,5-9 | C 07 C 87/44<br>C 07 D 295/02<br>C 07 D 275/06<br>A 01 N 43/80 |
| D,Y | EP-A-0 161 455 (BAYER)<br>* Patentansprüche *<br>----- | 1,5-9 | |
| | | | **RECHERCHIERTE SACHGEBIETE (Int. Cl.4)** |
| | | | C 07 C 87/00<br>C 07 D 295/00<br>C 07 D 275/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 26-02-1988 | MOREAU J.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)